# EUROPEAN PATENT APPLICATION

(11) **EP 2 443 995 A2**
(43) Date of publication of application: **25.04.2012**
(21) Application number: 11182710.1
(22) Date of filing: 26.09.2011
(51) Int. Cl.: A61B 5/0424

(54) **An ECG apparatus with lead-off detection**

(30) Priority: 21.10.2010 IE 20100679
(71) Applicant: Syncrophi Systems Ltd., Dangan Galway (IE)
(72) Inventor: Rotariu, Cosmin Nicolae, Galway (IE)
(74) Representative: Boyce, Conor

(57) **Abstract**

An ECG apparatus with lead-off detection, comprises right arm (R), left arm (L) and neutral (N) electrodes. A first amplifier circuit is connected to the L and R electrodes to provide an ECG signal on a first output and also separately to provide a signal related to the common mode noise. An inverting feedback amplifier feeds back an inverted common mode noise signal to the N electrode, the inverted common mode noise signal also being fed to a second amplifier circuit whose output changes state upon disconnection of one or both of the L and N electrodes.

## Description

The present invention relates to an electrocardiography (ECG) with lead-off detection.

Electrocardiography is the recording of the electrical activity of the heart. An ECG system employs chest leads. ECG machines usually have a lead off indicator to help identify a high impedance electrode. By having this, medical staff can reaffix the ECG electrode to its place and continue the measurements.

A lead off detection typically is done by injecting a AC high frequency signal through one of the leads and reading it on the other leads; see, for example, US2010081949, US2008004538 and US2008027338. If the high frequency signal is not detected on one of the other leads it means that the lead is off or the electrode-skin contact is very poor.

Another method is to use pull ups and set the leads to VCC. When all the leads are connected to the body the voltage will be less than VCC.

Another method, described in US2003083707, is to inject into the patient body a signal modulated with the carrier signal generated by the implanted pacer.

Still another method, described in US2003083584, is to sum all the leads and fit the signal into an instrumentation amplifier, then filter the signal by removing the mains frequency, dc offset and other high frequencies. After this the signal is compared with a reference voltage.

In summary, these known methods use a lot of hardware components, or involve a signal being injected into the patient body that can affect the functioning of, e.g., a pacemaker in some cases.

According to the present invention there is provided an ECG apparatus with lead-off detection, the apparatus comprising right arm (R), left arm (L) and neutral (N) electrodes, a first amplifier circuit connected to the L and R electrodes to provide an ECG signal on a first output and also separately to provide a signal related to the common mode noise, and an inverting feedback amplifier to feed back an inverted common mode noise signal to the N electrode, the inverted common mode noise signal also being fed to a second amplifier circuit whose output changes state upon disconnection of one or both of the L and N electrodes.

Preferably, said first amplifier circuit comprises an instrumentation amplifier.

Preferably, said second amplifier circuit comprises an operational amplifier having one input connected via a low pass filter to said inverted common mode noise signal and another input connected to a reference signal, said operational amplifier having an output which changes state upon disconnection of said neutral (N) electrode.

Alternatively or in addition, said second amplifier circuit comprises a comparator having one input connected to said inverted common mode noise signal and another input connected to said left arm (L) electrode, said comparator having an output which changes state upon disconnection of said L electrode.

In one embodiment, the apparatus further comprises a third operational amplifier having one input connected via a low pass filter to one of said left (L) or right (R) arm electrodes and another input connected to a reference signal, said third operational amplifier having an output which changes state upon disconnection of said one L or R electrode.

Preferably, in said embodiment, the apparatus further comprises a fourth operational amplifier having one input connected via a low pass filter to the other of said left (L) or right (R) arm electrode and another input connected to a reference signal, said fourth operational amplifier having an output which changes state upon disconnection of said other of said L or R electrode.

Preferably, said change of state occurring upon disconnection of an electrode comprises a step change which occurs relatively faster than a change of state of any other amplifier circuit in response to the disconnection of said electrode.

Embodiments of the invention are capable of detecting lead off using fewer components and without injecting any signal into the patient body, in contrast to the methods of the prior art referred to above.

Embodiments of the invention will now be described, by way of example, with reference to the accompanying drawings, in which:
Figure 1 is a circuit diagram of a first embodiment of the invention.
Figure 2 is a circuit diagram of a second embodiment of the invention.

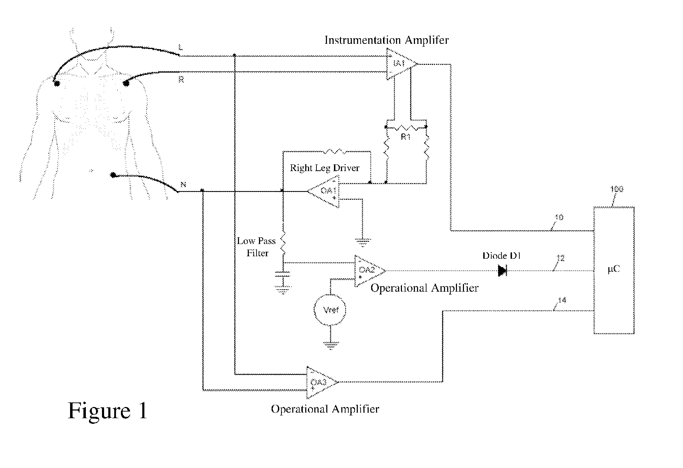

Referring to Figure 1, an ECG system has three leads L, R and N connected respectively to a left arm electrode, a right arm electrode and a neutral electrode. For convenience, each electrode will be referred to by its lead L, R or N. The L and R electrodes are connected to the positive and negative inputs respectively of an instrumentation amplifier lA1 and the ECG signal is provided at the output of lA1 which is connected as a first input 10 to a microcontroller 100. Separately, a voltage is developed across resistor R1 which is a measure of the common mode noise in the system. This common mode noise signal is inverted and fed back to the N electrode by an inverting feedback amplifier OA1. This arrangement is standard and does not require further description.

When all the electrodes are connected to the skin properly, the voltage on each electrode is close to 0. If one of the electrodes is off the skin, or making a poor connection with the skin, then the voltage on that electrode will rise slightly. This happens because the common mode noise from the skin referenced to the device is rising on the skin. This slight difference of potential can be detected.

In this first embodiment the lead-off detection is performed by two operational amplifiers OA2 and OA3, the amplifier OA2 being used as a comparator. The inverted common mode noise signal at the output of OA1 is fed to the negative input of the comparator OA2 via a passive low pass filter to eliminate the high frequencies that can toggle the comparator, and the positive input of OA2 is connected to a reference voltage Vref. The output of OA2 is connected as a second input 12 to the microcontroller 100 via a diode D1. The diode D1 maintains the signal used to trigger the interrupt on the microcontroller on the positive side in case of a dual supply comparator OA2. The L and N electrodes are connected respectively to the negative and positive inputs of OA3 whose output is connected as a third input 14 to the microcontroller.

When all the electrodes L, R and N are connected:
- the output 10 of lA1 is the ECG signal plus the DC offset which is set to VCC/2 (~1.5V, VCC is 3V).
- the output 12 of OA2 is high due to the fact that the voltage level on the negative input is lower than the reference voltage on the positive input.
- the output 14 of OA3 is high due to a pull up on the microcontroller.

When the electrode N is disconnected:
- the output 10 of lA1 stays at ~1.5V (VCC is 3V).
- the output 12 of OA2 output becomes low instantaneously
- the output 14 of OA3 output becomes low after about 10 seconds..

When the electrode R is disconnected:
- the output 10 of lA1 becomes low instantaneously (saturates on the negative side).
- the output 12 of OA2 becomes low after about 10 seconds.
- the output 14 of OA3 becomes low after about 10 seconds.

When the electrode L is disconnected:
- the output 10 of lA1 stays at ~1.5V (VCC is 3V).
- the output 12 of OA2 becomes low after about 10 seconds.
- the output 14 of OA3 becomes low instantaneously (saturates on the negative side).

The outputs 10, 12 and 14 are connected to an interrupt pin on the microcontroller 100 and when the signal goes low the interrupt is triggered and the change is detected.

In a case where two electrodes are disconnected the lead off condition will occur and will be reported until both leads are reconnected.

On reconnection the microcontroller 100 will recognize the electrodes connected to the body after about 10 seconds. When all the electrodes are detected as connected there is a calibration stage that lasts 12-15 seconds. In this calibration stage first there is a wait period of 7 seconds for data settling. This is the de-bouncing period. After this all buffers with data are cleared and the signal is passed through a calibration function to find and calculate parameters like gain and amplitude for the signal; this takes about 5 seconds. Next step is to clear again all the buffers, send calibration data to the server and start sending ECG data.

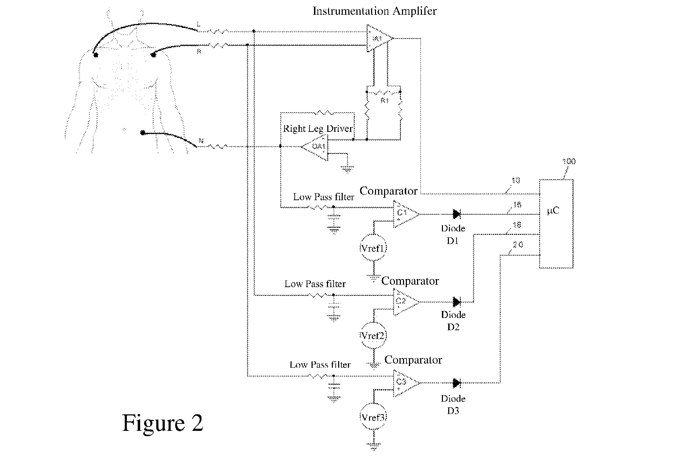

The second embodiment is shown in Figure 2, in which components which are the same or equivalent to those in Figure 1 have been given the same references and will not be described again.

The second embodiment used three comparators C1 to C3. The electrodes N, L and R are connected to the negative inputs of the comparators C1, C2 and C3 respectively, in each case via a passive low pass filter to eliminate the high frequencies that can toggle the comparator giving false readings . The positive inputs of each comparator C1, C2 and C3 are connected in each case to a respective reference voltage Vref1, Vref2 and Vref3. The output from each comparator C1 to C3 is connected via a respective diode D1 to D3 as a respective input 16, 18, 20 to the microcontroller 100, the diodes again serving to maintain the signal on the positive side in case of a dual supply comparator.

In case of an electrode disconnection the respective comparator output will toggle instantaneously to low and this triggers an interrupt on the microcontroller 100. Thus disconnection of electrode N will toggle the output of comparator C1 low, disconnection of electrode L will toggle the output of comparator C2 low, and disconnection of electrode R will toggle the output of comparator C3 low (it will be observed that the comparator C1 in Figure 2 operates in the same way as the comparator OA2 in Figurel).

The voltage references Vref1, Vref2 and Vref3 can be identical but in order to decrease the reaction time on lead off detection the reference voltages can be individually set for each comparator.

The comparator C2 of Figure 2 can be replaced with the comparator arrangement OA3 of Figure 1 but using the C2 configuration instead of OA3 helps to detect the lead reconnection faster.

By using the comparator configuration of Figure 2 the leads reconnection is detected faster than waiting for an operational amplifier to get out from its saturation state.

The invention is not limited to the embodiments described herein which may be modified or varied without departing from the scope of the invention.

## Claims

1. An ECG apparatus with lead-off detection, the apparatus comprising right arm (R), left arm (L) and neutral (N) electrodes, a first amplifier circuit connected to the L and R electrodes to provide an ECG signal on a first output and also separately to provide a signal related to the common mode noise, and an inverting feedback amplifier to feed back an inverted common mode noise signal to the N electrode, the inverted common mode noise signal also being fed to a second amplifier circuit whose output changes state upon disconnection of one or both of the L and N electrodes.

2. An ECG apparatus according to claim 1, wherein said first amplifier circuit comprises an instrumentation amplifier.

3. An ECG apparatus according to claim 1 or 2, wherein, said second amplifier circuit comprises an operational amplifier having one input connected via a low pass filter to said inverted common mode noise signal and another input connected to a reference signal, said operational amplifier having an output which changes state upon disconnection of said neutral (N) electrode.

4. An ECG apparatus according to any of claims 1 to 3, wherein, said second amplifier circuit comprises a comparator having one input connected to said inverted common mode noise signal and another input connected to said left arm (L) electrode, said comparator having an output which changes state upon disconnection of said L electrode.

5. An ECG apparatus according to any of claims 1 to 4, wherein the apparatus further comprises a third operational amplifier having one input connected via a low pass filter to one of said left (L) or right (R) arm electrodes and another input connected to a reference signal, said third operational amplifier having an output which changes state upon disconnection of said one L or R electrode.

6. An ECG apparatus according to claim 5, further comprising a fourth operational amplifier having one input connected via a low pass filter to the other of said left (L) or right (R) arm electrode and another input connected to a reference signal, said fourth operational amplifier having an output which changes state upon disconnection of said other of said L or R electrode.

7. An ECG apparatus according to claim 1, said change of state occurring upon disconnection of an electrode comprising a step change which occurs relatively faster than a change of state of any other amplifier circuit in response to the disconnection of said electrode.
